# EUROPEAN PATENT SPECIFICATION

(11) **EP 0 576 424 B1**
(45) Date of publication and mention of the grant of the patent: **24.04.1996**
(21) Application number: 91910715.1
(22) Date of filing: 14.06.1991
(51) Int. Cl.: C12P 19/14, C12N 9/96, C11D 3/386, C12N 9/24

(54) **ACTIVATION OF POLYSACCHARIDE HYDROLASE**
Aktivierung von Polysaccharide Hydrolase
ACTIVATION D'HYDROLASE DE POLYSACCHARIDE

(30) Priority: 14.06.1990 DK 1450/90; 27.03.1991 EP 91610023
(43) Date of publication of application: 05.01.1994
(73) Proprietor: NOVO NORDISK A/S, DK-2880 Bagsvaerd (DK)
(72) Inventor: ROSHOLM, Peter, DK-1370 Koebenhavn K (DK); SI, Joan, Qi, DK-2930 Klampenborg (DK); GYALOKAY, Otto, DK-2800 Lyngby (DK); WAGNER, Peter, DK-1719 Koebenhavn V (DK)
(86) International application number: DK9100158
(87) International publication number: WO9119807

(56) References cited:
- EP-A- 0 220 016
- WO-A-90/00593
- US-A- 3 860 484
- US-A- 4 176 079
- DIALOG INFORMATION SERVICES, File 351, WPI, 1981-1991; SHOWA DENKO KK, AN 7531906
- CHEMICAL ABSTRACTS, vol. 81, no. 7, 19 August 1974, Columbus, OH (US); A. IRIE et al., p. 175, no. 34870w
- CHEMICAL ABSTRACTS, vol. 76, no. 14, 03 April 1972, Columbus, OH (US); P. IONESCU-STOIAN et al., p. 281, no. 76402x
- CHEMICAL ABSTRACTS, vol. 98, no. 7, 14 February 1983, Columbus, OH (US); E.T. REESE et al., p. 304, no. 49392s

## Description

### TECHNICAL FIELD

This invention relates to a method of increasing the effect of a polysaccharide hydrolase.

### BACKGROUND ART

Polysaccharide hydrolases are enzymes classified in group EC 3.2.1 and having a polysaccharide as substrate. Examples are endo-glucanases such as α-amylase, cellulase and pullulanase, which promote endo-hydrolysis of the glucans starch, cellulose and pullulan, respectively.

Many industrial uses of polysaccharide hydrolases are known. Thus, it is known to use pullulanase together with a saccharifying enzyme in the saccharification of liquefied starch for production of glucose (dextrose) or maltose (e.g. EP 63,909) or for the hydrolysis of pullulan into maltotriose. It is known to use cellulase e.g. for colour clarification of textiles (EP 220,016).

### STATEMENT OF THE INVENTION

We have found that, surprisingly, addition of certain water-soluble polymers significantly increases the effect of polysaccharide hydrolases.

Accordingly, the invention provides a method of increasing the effect of a polysaccharide hydrolase, characterized by incorporation of a water-soluble polymer of one or more monomers selected from the group of vinyl pyrrolidone, vinyl alcohol, acrylamide and soluble acrylates into the reaction system in dissolved form.

Another aspect of the invention provides an enzymatic process using a polysaccharide hydrolase, characterized by the presence of the above water-soluble polymer.

Finally, the invention provides a polysaccharide hydrolase preparation, characterized by comprising said water-soluble polymer.

PVP has been used as a binder in granulation of enzymes (e.g. protease, glucose isomerase) without any effect on enzyme activity being reported (e.g. US 4,572,897), PVP has also been reported to improve the storage stability of enzymes (e.g. peroxidase, protease) (e.g. EP 303,062, WO 90/00593). V. Jancsik, J.Mol.Catal., 6 (1), 41-50 (1979) reports inhibition of one kind of aldolase by PVP. F.E. Prado et al., J.Biol.Chem. 260 (8), 4952-7 (1985) reports activation of invertase by PVP. J.R.L. Walker et al., Phytochemistry (Oxford), 19 (3), 373-8 (1980) reports that o-diphenol oxidase (catecholase) is inhibited by PVP, whereas p-diphenol oxidase (laccase) is unaffected by PVP. PVP has also been used in the recovery of enzymes without any effect on enzyme activity being reported (EP 262,651, EP 214,531, US 4,144,130, US 4,016,039).

However, the prior art gives no guidance to the skilled person, enabling him to predict activation of polysaccharide hydrolase by PVP.

### DETAILED DESCRIPTION OF THE INVENTION

### Polysaccharide hydrolase

The invention is applicable to activation of a polysaccharide hydrolase, i.e. an enzyme in group EC 3.2.1 according to Enzyme Nomenclature having polysaccharide as substrate. It is particularly applicable to activation of an endo-glucanase, i.e. an enzyme that effects endo-hydrolysis of a glucan (a polymer of glucose units), e.g. a cellulase, a pullulanase or an α-amylase.

Cellulase (EC 3.2.1.4) may be derived from *Humicola* (especially *H. lanuginosa,* US 4,435,307) or *Trichoderma* (e.g. *T. viride, T. reesei* or T. *koningii)* or may be prepared according to PCT/DK 90/00123. Pullulanase (EC 3.2.1.41) may be derived from *Bacillus,* particularly from *B. acidopullulyticus,* as described in EP 63,909. α-amylase (EC 3.2.1.1) may be derived from *Bacillus,* e.g. *B. amyloliquefaciens, B. licheniformis* or *B. stearothermophilus.*

The invention is also applicable to activation of inulinase (EC 3.2.1.7), derived e.g. from *Aspergillus,* especially *A. niger.*

### Enzymatic process

The invention serves to increase the enzymatic effect in a process using a polysaccharide hydrolase.

An example is a process using cellulase for colour clarification of textiles, as described in EP 220,016. In colour clarification, the enzyme and PVP can optionally be used together with a detergent.

Another example is saccharification of liquefied starch in the presence of pullulanase together with a saccharifying enzyme selected from the group of glucoamylase, β-amylase and maltogenic amylase. Another example is the use of pullulanase for hydrolysis of pullulan into maltotriose.

### Water-soluble polymer

The water-soluble polymer used in the invention is a polymer of one or more of the monomers vinyl pyrrolidone, vinyl alcohol, vinyl carboxylate (e.g. C₁-C₄ carboxylate such as acetate), acrylamide and acrylate (e.g. an alkali metal acrylate such as sodium acrylate). The polymer may be a homo-polymer or a copolymer, e.g. a block or random copolymer.

Examples of preferred polymers are PVP (polyvinyl pyrrolidone), PVA (polyvinyl alcohol), polyacrylate, and copolymers of vinyl pyrrolidone with acrylamide or vinyl alcohol.

PVP used in the invention preferably has an average molecular weight of 5,000 - 1,000,000, particularly 5,000-50,000. The amount of PVP is preferably 0.005 - 5% in the reaction medium or 0.03-3 g PVP/kg dry solids at process conditions. In the enzyme preparation the amount of PVP is preferably 0.005 - 10%, particularly 0.1 - 5% (all percentages by weight). In the case of pullulanase, the amount of PVP is preferably 5*10⁻⁴ . - 5*10⁻ g PVP/PUN.

The amount of polymer is preferably 0.5-100 mg/l, e.g. 0.5-50 mg/l, in the reaction system or 0.005-10%, e.g. 0.1-5%, in the enzyme preparation.

PVA used in the invention is preferably a partially hydrolysed polyvinyl ester of a lower (e.g. C₁-C₄) carboxylic acid, especially polyvinyl acetate, which has a degree of hydrolysis above 75%, particularly above 90%, e.g. above 95%. The PVA preferably has a molecular weight of 5,000 - 200,000, e.g. 20,000 - 200,000, particularly 50,000 - 100,000.

The above-mentioned copolymers are preferably random copolymers having a ratio of vinyl pyrrolidone to vinyl alcohol or acrylamide in the range 1:10 to 10:1 (on molar basis).

### Enzyme preparation

The enzyme preparation of the invention may be provided in the form of a stabilized liquid or non-dusting granulate, suited for storage and shipping, or it may be an aqueous solution, ready for use.

Non-dusting granulates may be produced, e.g., as disclosed in US 4,106,991 and 4,661,452 (both to Novo Industri A/S) and may optionally be coated by methods known in the art.

Liquid enzyme preparations may, for instance, be stabilized by adding a polyol such as propylene glycol, a sugar alcohol or a carbohydrate, e.g. sucrose in an amount of e.g. 20-80%, preferably above 50%. Lactic acid may also be used as a stabilizer, and the liquid may further contain a preservative such as sodium benzoate, typically 0.1-0.5%. A liquid pullulanase preparation according to the invention may further contain sodium sulphite or bisulphite corresponding to 0.1-2% as SO₂.

Depending on the intended use, the enzyme may be mixed with other active ingredients. Thus, pullulanase may be used as a mixed enzyme preparation containing pullulanase together with the above-mentioned saccharification enzyme. Cellulase together with PVP may be incorporated into a detergent composition, which may be liquid or granular.

### Determination of cellulase activity (CEVU)

A substrate solution is prepared, containing 33.3 g/l CMC (Hercules 7 LFD) in 0.1 M Tris buffer at pH 9.0. The enzyme sample to be analyzed is dissolved in the same buffer. 10 ml substrate solution and 0.5 ml enzyme solution are mixed and transferred to a viscosimeter (e.g. Haake VT 181, NV sensor, 181 rpm) thermostated at 40°C. One Cellulase Viscosity Unit (CEVU) is defined as the amount of enzyme that reduces the viscosity to one half under these conditions.

### Determination of pullulanase activity (PUN)

This unit is defined in EP 63,909.

### EXAMPLES

### EXAMPLE 1

### Colour clarification of textile with Humicola, cellulase and PVP

Black cotton swatches were treated by 3 repeated washings in a Terg-O-Tometer^{™} each followed by a rinsing and drying process. Washing conditions were as follows:

| | |
|---|---|
| Liquid volume: | 800 ml |
| Agitation | 100 movements/min. |
| Washing time: | 30 min. |
| Washing temp: | 40°C |
| pH | 7.0 |
| Water hardness: | 9 dH |
| Textile: | 100% cotton |
| Amount of textile: | 2 swatches/800 ml, each 10*15 cm |
| Rinsing time: | 30 min in running tap water |
| Drying: | line-drying, room temp (16 hours) |

The detergent was a commercial US liquid detergent, used at a dosage of 2 g/l. Varying amounts of PVP (molecular weight 30,000) was added to the detergent.

Cellulase from *H. insolens* prepared according to US 4,435,307 was added to the detergent solution immediately before washing at a dosage of 0, 50 or 100 CEVU/l.

The surface of the swatches was analyzed by measuring reflected light. In this measurement, white light is projected onto the surface and the reflection/remission is detected at 16 wavelengths by means of an "Elrepho 2000" Datacolor Schweiz apparatus. The results of these detections are processed into Hunter coordinates of which the L-coordinate represents the grey scale value (100: black, 0: white).

The effect of the enzyme is removal of damaged cellulose fibers causing a grey/worn look, and by measuring the grey scale value a quantitative expression for the enzyme treatment is obtained. The results are expressed as delta L in relation to the surface treated without cellulase and PVP.

Detergent I:

| | 0 CEVU/I | 50 CEVU/I | 100 CEVU/I |
|---|---|---|---|
| 0% PVP | 0 | 1.14 | 1.38 |
| 0.1% PVP | 0.25 | 1.49 | 1.8 |
| 0.5% PVP | 0.17 | 1.64 | 1.84 |
| 1.0% PVP | 0.11 | 1.82 | 1.87 |

It is seen that the colour clarifying effect of the cellulase is increased by addition of PVP.

### EXAMPLE 2

### Colour clarification of textile with cellulase and PVA

This experiment was made in the same manner as in Example 1, but using a buffer system instead of detergent and PVA instead of PVP. The PVA had an average molecular weight of 72,000 and a degree of hydrolysis of 98%. The buffer system contained 5% (w/w) triethanol amine, 10% ethanol and 85% H₂O. A dosage of 2 g/l was used.

Results (expressed in Delta L as in Example 1):

| | 0 CEVU/I | 10 CEVU/I | 25 CEVU/I |
|---|---|---|---|
| 0mg/l PVA | 0.0 | 0.21 | 0.53 |
| 100 mg/l PVA | -0.21 | 0.71 | 1.08 |

### EXAMPLE 3

### Activation of cellulase with PVA/PVP copolymer

This experiment was made in the same manner as in Example 2, but using a random copolymer of vinyl alcohol and vinyl pyrrolidone instead of PVA. Results:

| | 0 CEVU/I | 10 CEVU/I | 25 CEVU/I |
|---|---|---|---|
| 0 mg/l PVA/PVP | 0.0 | 0.21 | 0.53 |
| 100 mg/l PVA/PVP | 0.15 | 0.98 | 1.08 |

### EXAMPLE 4

### Activation of cellulase with PVP/acrvlamide copolymer

This experiment was made in the same manner as in Example 2, but using a random copolymer of vinyl pyrrolidone and acrylamide instead of PVA. Results:

| | 0 CEVU/I | 10 CEVU/I | 25 CEVU/I |
|---|---|---|---|
| 0 mg/l copolymer | 0.0 | 0.43 | 0.49 |
| 100 mg/l copolymer | 0.06 | 0.44 | 0.99 |

### EXAMPLE 5

### Activation of Trichoderma cellulase in CMC hydrolysis

Cellulase from *T*. *reesei* having an activity of 675 EGU/g (cellulase activity unit described in WO 91/07542) was tested with and without addition of PVP in hydrolysis of carboxymethyl cellulose (CMC). The amount of PVP was expressed as % by weight relative to the enzyme preparation.

A substrate solution was prepared, containing 34,0 g/l CMC (Hercules 7 LFD) in 0.1 M phosphate buffer at pH 6.0. The enzyme sample to be analyzed was dissolved in the same buffer. 5 ml substrate solution and 0.15 ml enzyme solution were mixed and transferred to a vibration viscosimeter (e.g. MIVI 3000 from Sofraser, France), thermostated at 40°C. The relative activity was calculated from the amount of enzyme that reduces the viscosity to one half under these conditions; the result without addition of PVP was taken as 100. The amount of enzyme sample was 0.01-0.02 EGU/ml in the reaction mixture.

Results:

| % PVP | Relative activity |
|---|---|
| 0 (reference) | 100 |
| 1% | 103 |
| 3% | 110 |

### EXAMPLE 6

### Activation of pullulanase in pullulan hydrolysis

A liquid pullulanase preparation was made according to EP 63,909. The preparation contained a pullulanase activity of 180 PUN/g and 50% sucrose.

Varying amounts of PVP (MW 15,000) were added to this preparation, and the apparent pullulanase activity was determined by the PUN method. PVP content is given as % by weight in the preparation. Results:

| % PVP | PUN/g |
|---|---|
| 0 (reference) | 180 |
| 0.1 | 190 |
| 0.5 | 220 |
| 1.0 | 240 |

The results clearly show increasing activation up to 33% increase at 1.0% PVP.

### EXAMPLE 7

### Activation of pullulanase in saccharification

In this example, liquefied starch was saccharified with glucoamylase and pullulanase containing PVP. The experiments were run essentially as in EP 63,909.

The pullulanase samples of Example 6 were used and were dosed according to the apparent activity given in Example 6, i.e. correspondingly less of the preparations activated by PVP. The enzyme dosages were 0.18 AG/g of glucoamylase and 0.06 PUN/g of pullulanase. Saccharification conditions were 60°C, initial pH 4.3. Analyses by HPLC were made after 24, 48, 72 and 96 hours. The maximum % DP1 was as follows:

| % PVP | max. % DP1 | Max reached after hours |
|---|---|---|
| 0 | 96.4 | 72 |
| 0.1 | 96.4 | 72 |
| 1.0 | 96.4 | 72 |

Identical results were obtained, indicating that the same degree of activation is found in saccharification of hydrolyzed starch as in pullulan hydrolysis.

### EXAMPLE 8

### Activation of inulinase

Inulinase from *A*. *niger* was tested with and without addition of PVP in hydrolysis of inulin. The amount of PVP was expressed as % by weight relative to the enzyme preparation.

The conditions used for inulin hydrolysis were 50 g/l inulin (Inulin Sigma 1 3754) in 0.025 citrate buffer at pH 4.70, 40°C, 30 minutes reaction time. The concentration of reducing carbohydrates produced during the incubation was determined by means of the Somogyi Nelson reaction. The reference sample without PVP produced 3107 mol reducing carbohydrate (calculated as glucose) per g of enzyme preparation, and this was taken as 100%. Results:

| % PVP in enzyme preparation | Relative inulin hydrolysis |
|---|---|
| 0 (reference) | 100 |
| 1% | 107 |
| 3% | 111 |

## Claims

1. A method of increasing the effect of a cellulase, a pullulanase or an inulinase, characterized by incorporating a water-soluble polymer of one or more monomers selected from the group of vinyl pyrrolidone, vinyl alcohol, vinyl carboxylate, acrylamide and soluble acrylates into the reaction system in dissolved form.

2. A method according to Claim 1 wherein the concentration of said polymer in the reaction system is 0.5-100 mg/l.

3. A method according to Claim 1 or 2, wherein said polymer is polyvinyl pyrrolidone (PVP), polyvinyl alcohol (PVA), polyacrylate or a copolymer of vinyl pyrrolidone with vinyl alcohol or acrylamide.

4. A method according to Claim 3, wherein said PVP has an average molecular weight of 5,000 - 1,000,000, preferably 5,000-50,000.

5. A method according to Claim 3, wherein said PVA is a partially hydrolyzed polyvinyl ester of a C₁₋₄ carboxylic acid having a degree of hydrolysis above 75% and an average molecular weight of 5,000 - 200,000.

6. An enzymatic process using a cellulase, a pullulanase or an inulinase, characterized by the presence of a water-soluble polymer of one or more monomers selected from the group of vinyl pyrrolidone, vinyl alcohol, vinyl carboxylate, acrylamide and soluble acrylates in dissolved form.

7. A process according to Claim 6 using cellulase for colour clarification of cotton textile.

8. A process according to Claim 6 using pullulanase for the saccharification of liquefied starch together with a saccharifying enzyme selected from the group of glucoamylase, β-amylase and maltogenic amylase.

9. A pullulanase or inulinase preparation, characterized by comprising a water-soluble polymer of one or more monomers selected from the group of vinyl pyrrolidone, vinyl alcohol, acrylamide and soluble acrylates.

10. A mixed pullulanase preparation according to Claim 9, further comprising a saccharifying enzyme selected from the group of glucoamylase, β-amylase and maltogenic amylase.

11. A preparation according to Claim 9 or 10 in the form of a non-dusting granulate or a stabilized liquid.

12. A liquid cellulase preparation, characterized by comprising a water-soluble polymer of one or more monomers selected from the group of vinyl pyrrolidone, vinyl alcohol, acrylamide and soluble acrylates in dissolved form.

13. A cellulase preparation, characterized by comprising a water-soluble polymer of one or more monomers selected from the group of vinyl pyrrolidone, acrylamide and soluble acrylates.

14. A detergent composition comprising a cellulase preparation according to Claim 12 or 13.

15. A preparation according to any of Claims 9-12, comprising 0.005 - 10%, preferably 0.1 - 5% of said polymer, by weight.

## Patentansprüche

1. Verfahren zur Erhöhung der Wirkung einer Cellulase, einer Pullulanase oder einer Inulinase, gekennzeichnet durch Einverleiben eines wasserlöslichen Polymeren aus einem oder mehreren Monomeren, ausgewählt aus der Gruppe Vinylpyrrolidon, Vinylalkohol, Vinylcarboxylat, Acrylamid und löslichen Acrylaten, in das Reaktionssystem in gelöster Form.

2. Verfahren nach Anspruch 1, wobei die Konzentation des Polymeren im Reaktionssystem 0,5-100 mg/l beträgt.

3. Verfahren nach Anspruch 1 oder 2, wobei das Polymere Polyvinylpyrrolidon (PVP), Polyvinylalkohol (PVA), Polyacrylat oder ein Copolymeres von Vinylpyrrolidon mit Vinylalkohol oder Acrylamid ist.

4. Verfahren nach Anspruch 3, wobei das PVP ein durchschnittliches Molekulargewicht von 5.000-1.000.000, vorzugsweise 5.000-50.000 aufweist.

5. Verfahren nach Anspruch 3, wobei das PVA ein teilweise hydrolysierter Polyvinylester einer C₁₋₄-Carbonsäure ist mit einem Hydrolysegrad oberhalb 75 % und einem durchschnittlichen Molekulargewicht von 5.000-200.000.

6. Enzymatisches Verfahren unter Verwendung einer Cellulase, einer Pullulanase, oder einer Inulinase, gekennzeichnet durch die Anwesenheit eines wasserlöslichen Polymeren von einem oder mehreren Monomeren, ausgewählt aus der Gruppe Vinylpyrrolidon, Vinylalkohol, Vinylcarboxylat, Acrylamid und löslichen Acrylaten in gelöster Form.

7. Verfahren nach Anspruch 6, wobei Cellulase zur Farbklarifikation von Baumwolltextilien verwendet wird.

8. Verfahren nach Anspruch 6, wobei Pullulanase zur Verzuckerung von verflüssigter Stärke zusammen mit einem verzuckernden Enzym verwendet wird, ausgewählt aus der Gruppe Glucoamylase, β-Amylase und maltogene Amylase.

9. Pullulanase- oder Inulinasepräparat, dadurch gekennzeichnet, daß es ein wasserlösliches Polymeres aus einem oder mehreren Monomeren umfaßt, ausgewählt aus der Gruppe Vinylpyrrolidon, Vinylalkohol, Acrylamid und lösliche Acrylate.

10. Gemischtes Pullulanasepräparat gemäß Anspruch 9, weiterhin umfassend ein verzuckerndes Enzym, ausgewählt aus der Gruppe Glucoamylase, β-Amylase und maltogene Amylase.

11. Präparat gemäß Anspruch 9 oder 10 in Form eines nicht-staubenden Granulats oder einer stabilisierten Flüssgkeit.

12. Flüssiges Cellulasepräparat, dadurch gekennzeichnet, daß es ein wasserlösliches Polymeres aus einem oder mehreren Monomeren umfaßt, ausgewählt aus der Gruppe Vinylpyrrolidon, Vinylalkohol, Acrylamid und lösliche Acrylate in gelöster Form.

13. Cellulasepräparat, dadurch gekennzeichnet, daß es ein wasserlösliches Polymeres aus einem oder mehreren Monomeren umfaßt, ausgewählt aus der Gruppe Vinylpyrrolidon, Acrylamid und lösliche Acrylate.

14. Detergenszusammensetzung, umfassend ein Cellulasepräparat gemäß Anspruch 12 oder 13.

15. Präparat gemäß einem der Ansprüche 9-12, umfassend 0,005-10 Gew.-%, vorzugsweise 0,1-5 Gew.-% des Polymeren.

## Revendications

1. Procédé destiné à augmenter l'effet d'une cellulase, d'une pullulanase ou d'une inulinase, caractérisé en ce qu'il comporte un polymère soluble à l'eau constitué d'un ou de plusieurs monomères sélectionnés parmi le groupe constitué de vinyle pyrrolidone, d'alcool vinylique, de carboxylate vinylique, d'acrylamide et d'acrylates solubles, dans le système de réaction sous une forme dissoute.

2. Procédé selon la revendication 1, dans lequel la concentration dudit polymère dans le système de réaction est située entre 0,5 et 100 mg/l.

3. Procédé selon les revendications 1 ou 2, dans lequel ledit polymère est une polyvinyle pyrrolidone (PVP), un alcool polyvinylique (PVA), un polyacrylate ou un copolymère de vinyle pyrrolidone avec un alcool vinylique ou une acrylamide.

4. Procédé selon la revendication 3, dans lequel ladite PVP a un poids moléculaire moyen situé entre 5 000 et 1 000 000, de préférence entre 5 000 et 50 000.

5. Procédé selon la revendication 3, dans lequel ledit PVA est un ester polyvinylique, hydrolysé de manière partielle, d'un acide carboxylique de type C₁₋₄ ayant un degré d'hydrolyse supérieur à 75% et un poids moléculaire moyen situé entre 5 000 et 200 000.

6. Processus enzymatique utilisant une cellulase, une pullulanase ou une inulinase, caractérisé par la présence d'un polymère soluble à l'eau constitué d'un ou de plusieurs monomères sélectionnés parmi le groupe constitué de vinyle pyrrolidone, d'alcool vinylique, de carboxylate vinylique, d'acrylamide et d'acrylates solubles, sous une forme dissoute.

7. Processus selon la revendication 6, utilisant une cellulase pour la clarification d'un textile en coton.

8. Processus selon la revendication 6, utilisant une pullulanase pour la saccharification d'amidon liquéfié associée à une enzyme de saccharification sélectionnée par le groupe constitué de la glucoamylase, la β-amylase et l'amylase maltogène.

9. Préparation de pullulanase ou d'inulinase, caractérisée en ce qu'elle comporte un polymère soluble à l'eau constitué d'un ou de plusieurs monomères sélectionnés parmi le groupe constitué de vinyle pyrrolidone, d'alcool vinyliques, d'acrylamide et d'acrylates solubles.

10. Préparation de pullulanase mixte selon la revendication 9, comportant de plus une enzyme de saccharification sélectionnée parmi le groupe constitué de la glucoamylase, la β-amylase et l'amylase maltogène.

11. Préparation selon les revendications 9 ou 10, sous la forme d'un granulé non-poussiéreux ou d'un liquide stabilisé.

12. Préparation de cellulase liquide, caractérisée en ce qu'elle comporte un polymère soluble à l'eau constitué d'un ou de plusieurs monomères sélectionnés parmi le groupe constitué de vinyle pyrrolidone, d'alcool vinylique, d'acrylamide et d'acrylates solubles, sous une forme dissoute.

13. Préparation de cellulase, caractérisée en ce qu'elle comporte un polymère soluble à l'eau constitué d'un ou de plusieurs monomères sélectionnés parmi le groupe constitué de vinyle pyrrolidone, d'acrylamide et d'acrylates solubles.

14. Composition de détergent comportant une préparation de cellulase selon les revendications 12 ou 13.

15. Préparation selon l'une quelconque des revendications 9 à 12, comportant 0,005 à 10%, de préférence 0,1 à 5% dudit polymère, en poids.
